# EUROPÄISCHE PATENTANMELDUNG

(11) **EP 1 905 413 A1**
(43) Veröffentlichungstag der Anmeldung: **02.04.2008**
(21) Anmeldenummer: 06121333.6
(22) Anmeldetag: 27.09.2006
(51) Int. Cl.: A61K 6/083, C08F 4/72

(54) **Polymerisierbare Zusammensetzungen mit Acylgermanen als Initiatoren**

(71) Anmelder: Ivoclar Vivadent AG, 9494 Schaan (LI)
(72) Erfinder: Moszner, Norbert, 9495, Triesen (LI); Salz, Ulrich, 88131, Lindau (DE); Fischer, Urs-Karl, 9320, Arbon (CH); Liska, Robert, 1200, Wien (AT); Gruber, Heinrich, 1190, Wien (AT); Ganster, Beate, 1190, Wien (AT)
(74) Vertreter: UEXKÜLL & STOLBERG

(57) **Zusammenfassung**

Zusammensetzung mit mindestens einem polymerisierbaren Bindemittel und einem Polymerisationsinitiator, die mindestens ein Acylgerman gemäß der allgemeinen Formel (I) enthält, 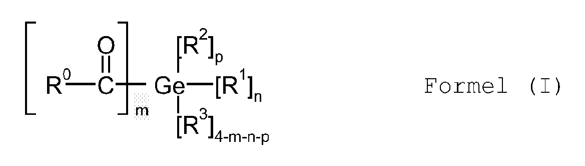 in der R⁰ ein substituierter oder unsubstituierter C₁₋₁₈-Alkylrest oder eine Acylgruppe ist; R¹ und R² H, eine Acylgruppe sind oder eine der für R³ angegebenen Bedeutungen haben; R³ ein verzweigter oder linearer C₁₋₁₈-Alkyl-Rest, der unsubstituiert oder einfach oder mehrfach substituiert oder durch -O-, -NH-, -NR-, -S- oder andere Gruppen unterbrochen sein kann, Trimethylsilyl, Hal-(CH₃)₂Si-[OSi(CH₃)₂]ᵣ-, (CH₃)₃Si-[OSi(CH₃)₂]ᵣ-, -COOH, -COO-R¹⁰, -CO-NP¹¹R¹², -CO-Vinyl, -CO-Phenyl, Phenyl-C₁₋₄-Alkyl, Phenyl, Naphthyl oder Biphenyl, C₅₋₁₂-Cycloalkyl, ein 5- oder 6-gliedriger O-, S- oder N-haltiger heterocyclischer Ring, Halogen, OH, ein aromatischer C₆₋₃₀-Rest, der durch substituiert oder unsubstituiert und/oder durch O, S oder -NR- unterbrochen sein kann, oder ein verzweigter, zyklischer oder linearer C₁₋₂₀-Alkyl-, -Alkenyl-, -Alkoxy- oder -Alkenoxy-Rest ist; m 1, 2 oder 3 ist; n 0 oder 1 ist und p 0 oder 1 ist; sowie die Verwendung von Acylgermanen der Formel (I) beispielsweise als Initiator für die radikalische Polymerisation.

## Beschreibung

Die vorliegende Erfindung betrifft polymerisierbare Zusammensetzungen, die ein Acylgerman und insbesondere ein Mono- oder Bisacylgerman als Polymerisationsinitiator enthalten. Die Zusammensetzungen eignen sich besonders zur Herstellung von Adhäsiven, Beschichtungen, Zementen, Kompositen, von Formteilen und insbesondere von Dentalwerkstoffen.

Für die Aushärtung von polymerisationsfähigen Harzen spielt der eingesetzte Initiator eine entscheidende Rolle. Photoinitiatoren absorbieren bei Bestrahlung UV- oder sichtbares Licht und bilden die polymerisationsauslösenden Spezies. Im Fall der radikalischen Polymerisation handelt es sich dabei um freie Radikale. Basierend auf dem chemischen Mechanismus der Radikalbildung werden die Photoinitiatoren in zwei Klassen eingeteilt.

Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion wobei der Photoinitiator im angeregten Zustand mit einem zweiten Molekül, dem Coinitiator, reagiert und durch Elektronen- und Protonentransfer oder direkte Wasserstoffabstraktion die polymerisationsauslösenden Radikale bildet. Für die UV-Lichthärtung werden Typ-I- und Typ-II-Photoinitiatoren eingesetzt, für den sichtbaren Lichtbereich kommen bisher nahezu ausschliesslich Typ-II-Photoinitiatoren zum Einsatz.

Die UV-Härtung zeichnet sich durch eine hohe Reaktionsgeschwindigkeit aus und wird häufig für die Beschichtungen von unterschiedlichen Substraten wie z.B. Holz, Metall oder Glas eingesetzt. So wird zum Beispiel in EP 1 247 843 ein UV-härtendes Beschichtungsmaterial beschrieben, bei dem Typ-I-Photoinitiatoren wie Diethoxyphenylacetophenon oder Acylphosphinoxide zum Einsatz kommen.

WO 01/51533 beschreibt eine UV-härtendes Holzbeschichtungsmaterial bei dem ebenfalls Acylphosphinoxide, α-Hydroxyalkylphenone oder α-Dialkoxyacetophenone als Photoinitiator Anwendung finden. Bedingt durch die geringe Wellenlänge des UV-Lichts lassen sich mit der UV-Härtung vor allem transparente Beschichtungen mit geringer Schichtstärke härten, bei starker Einfärbung oder Pigmentierung und größeren Schichtstärken stößt man jedoch an das Limit der UV-Härtung, derartige photopolyreaktionsfähigen Harze härten mit UV-Licht nur unvollständig aus. Bei pigmentierten Zusammensetzungen muß außerdem ein Absorptionsbereich für den Photoinitiator gefunden werden, in dem das Pigment nur schwach absorbiert.

Werden größere Durchhärtungstiefen benötigt, wie zum Beispiel bei der Aushärtung von lichthärtenden Dentalfüllungsmaterialien, so wird in der Regel mit sichtbarem Licht bestrahlt. Das dafür am häufigsten eingesetzte Photoinitiatorsystem ist die Kombination aus einem α-Diketon mit einem Amincoinitiator wie sie in GB 1 408 265 beschrieben ist.

Dentalzusammensetzungen, in denen dieses Photoinitiatorsystem eingesetzt wird, werden z.B. in der US 4,457,818 oder US 4,525,256 offenbart, wobei vorzugsweise Campherchinon als α-Diketon eingesetzt wird. Campherchinon hat ein Absorptionsmaximum bei einer Wellenlänge von 468 nm. Dadurch zeigt Campherchinon eine starke Gelbfärbung, mit dem Nachteil, daß mit Campherchinon/Amin initiierte Materialien nach der Aushärtung einen deutlichen Gelbstich aufweisen. Dies ist vor allem bei hellen Weißtönen des auspolymerisierten Materials sehr nachteilig.

Der Erfindung liegt die Aufgabe zugrunde, Polymerisationsinitiatoren zur Verfügung zu stellen, die mit sichtbarem Licht aktiviert werden können und die eine hohe Durchhärtungstiefe des zu härtenden Materials ergeben. Die Initiatoren sollen in geringer Konzentration wirksam sein und eine schnelle Aushärtung des zu härtenden Materials ermöglichen. Außerdem sollen sie nicht zu Verfärbungen des Materials führen.

Erfindungsgemäß wird diese Aufgabe durch Zusammensetzungen mit mindestens einem polymerisierbaren Bindemittel und mindestens einem Polymerisationsinitiator gelöst, die mindestens ein Acylgerman gemäß der allgemeinen Formel (I) enthalten, 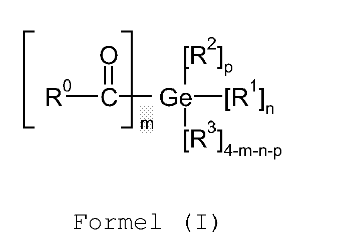 in der
- R°: C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰, -OCO-Hal, -COO-R¹⁰, -CH=CH-CO-OR¹⁰, -N(R¹¹)-CO-R¹⁰, -N(R¹¹)-CO-Hal, -C(C₁₋₄-Alkyl)=C(C₁₋₄-Alkyl)-CO-OR¹⁰, -CO-NR¹¹R¹², -CH=CH-Phenyl, -C(C₁₋₄-Alkyl)=C(C₁₋₄-Alkyl)-Phenyl, C₃₋₁₂-Cycloalkyl, C₂₋₁₈-Alkenyl, Phenyl-C₁₋₄-Alkyl, Phenyl, Naphtyl, Antryl, Biphenyl, ein 5- oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 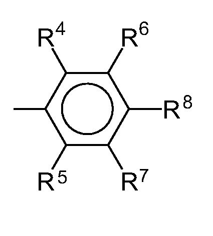 ist, wobei
- R¹⁰: H, C₁₋₁₈-Alkyl, C₂₋₁₈-Alkenyl, C₂₋₁₈-Alkenyl, das durch ein oder mehrere Sauerstoffatome unterbrochen ist, ein Di-, Tri-, Tetra- oder Polyethylenglycolrest, C₃₋₁₂-Cycloalkyl, Tetrahydropyran-2-yl, Phenyl-C₁₋₄-Alkylen, Phenyl-C₁₋₄-Alkenylen, C₁₋₆-Alkyl, das unsubstituiert oder durch Halogen, Cyclohexyl, Cylopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
- R¹¹, R¹²: unabhängig voneinander H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloakyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocyclischen Ring, der seinerseits mit einem aliphatischen oder aromatischen Ring anelliert sein kann,
- R¹, R²: unabhängig voneinander 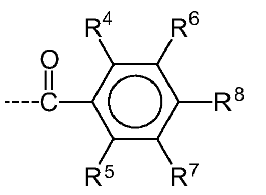 oder H sind, oder eine der für R³ angegebenen Bedeutungen haben; wobei
- R⁴, R⁵: unabhängig voneinander jeweils H, Halogen, ein verzweigter oder linearer C₁₋₆-Alkyl- oder -O-C₁₋₆-Alkylrest sind;
- R⁶, R⁷, R⁸: unabhängig voneinander jeweils H, Halogen, ein verzweigter, zyklischer oder vorzugsweise linearer C₁₋₂₀-Alkyl-, -Alkenyl-, -Alkyloxy- oder -Alkenoxy-Rest sind, der durch ein oder mehrere O, S oder -NR'- unterbrochen und durch eine oder mehrere polymerisationsfähige Gruppen und/oder Reste R⁹ substituiert sein kann, wobei R' H, Halogen, ein verzweigter, zyklischer oder vorzugsweise linearer C₁₋₂₀-Alkyl-, -Alkenyl-, -Alkyloxy- oder -Alkenoxy-Rest ist;
- R³: ein verzweigter oder vorzugsweiser linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist: Halogen, CN, 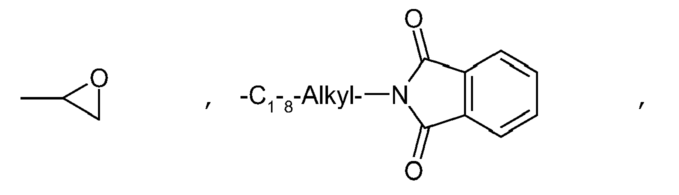 -OR¹⁰, -SR¹⁰, -OCO-R¹⁰, -COO-R¹⁰, -CH=CH-CO-OR¹⁰, -C(C₁₋₄-Alkyl)=C (C₁₋₄-Alkyl) -CO-OR¹⁰, -CO-R ¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N (R¹¹) -CO-R¹⁰, -N (R¹¹) -COO-R¹⁰, -N (R¹¹) -CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, - C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein 5- oder 6-gliedriger O-, S- oder N-haltiger heterocyclischer Ring, Benzophenonyl, Thisanthonyl, wobei
- R¹³: C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
- R¹⁴, R¹⁵, R¹⁶: unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁-₈-Alkyl, C₂₋₈-Alkenyl, C₇₋₉-Phenylalkyl, -O-C₁₋₈-Alkyl oder Phenyl sind, und
wobei R¹⁰, R¹¹ und R¹² wie oben definiert sind;
oder
- R³: ist ein verzweigter oder vorzugsweiser linearer C₂₋₁₈-Alkyl-Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹)-CO-R¹⁰, -N (R¹¹) -COO-R¹⁰, -N(R¹¹)-CO-NR¹¹R¹², -N (R¹¹) - CO-Hal, -CO-NR¹¹R¹², -SO₂-R¹⁰, -SO₂-OR¹⁰, -SO₂-NR¹¹R¹², - PO (OC₁₋₈-Alkyl) ₂, -SiR¹⁴R¹⁵R¹⁶, Phenyl-C₁₋₄-Alkyl, Phenyl, C₅₋₁₂-Cycloalkyl;
wobei R¹⁰ R¹¹, R¹² R¹⁴, R¹⁵ und R¹⁶ wie oben definiert sind;
oder
- R³: ist ein verzweigter oder vorzugsweiser linearer C₂₋₁₈-Alkyl-Rest oder ein C₂₋₁₈-Alkylen-Rest, der einfach oder mehrfach durch -CO-, -COO-, -OCO-, -OCOO-, -CO-N(R¹²)-, -N(N¹²)-CO-, -N(R¹²)-CO-N(R¹²)-, -N(R¹²)-COO-, -COO-C₁₋₆-Alkylen, -COS-C₁₋₁₈-Alkylen, -SO₂-, -SO₂-O-, -SO₂-N(R¹²)-, -(CH₃)₂Si[OSi (CH₃)₂]_{q}-, mit q = 1 bis 6; Phenyl-C₁₋₄-Alkylen, Phenylen, Naphthylen, Biphenylen, C₅₋₁₂-Cycloakylen oder einen 5- oder 6-gliedrigen O-, S- oder N-haltigen heterocyclischen Ring unterbrochen ist;
wobei R¹² wie oben definiert ist;
oder
- R³: ist Trimethylsilyl, Hal-(CH₃)₂Si-[OSi(CH₃)₂]ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
- R³: ist Phenyl-C₁₋₄-Alkyl, Phenyl, Naphthyl oder Biphenyl, C₅₋₁₂-Cycloalkyl oder ein 5- oder 6-gliedriger O-, S-oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind und
- m: 1, 2 oder 3 ist,
- n: 0 oder 1 ist,
- p: 0 oder 1 ist;
oder vorzugsweise
- R³: ist Halogen, OH, ein aromatischer C₆₋₃₀-Rest, der durch einen verzweigten, zyklischen oder vorzugsweise linearen C₁₋₂₀-Alkyl-, -Alkenyl-, Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, zyklischer oder vorzugsweise linearer C₁₋₂₀-Alkyl-, -Alkenyl-, -Alkoxy- oder -Alkenoxy-Rest ist, der ein- oder mehrfach durch O, S oder - NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x= 1 bis 20, -[Si(CH₃)₂]_{y}-CH₃ mit y = 1 bis 20 ist, und
R²⁰ H, Halogen, ein verzweigter, zyklischer oder vorzugsweise linearer C₁₋₂₀-Alkyl-, -Alkenyl-, -Alkyloxy- oder -Alkenoxy-Rest ist.

Bei den Acylgermanen der Formel (I) handelt es sich um Mono-, Bis-, oder Triacylgermane, wobei Mono- und Bisacylgermane bevorzugt sind.

Erfindungsgemäß besonders bevorzugt sind Verbindungen gemäß der folgenden Formel (II) 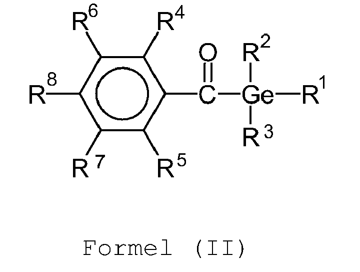 in der
- R¹, R²: unabhängig voneinander 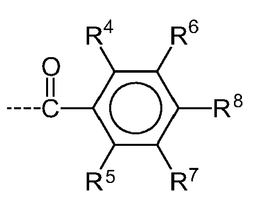 oder H sind, oder eine der für R³ angegebenen Bedeutungen haben;
- R³: Halogen, OH, ein aromatischer C₆₋₃₀-Rest, der durch einen verzweigten, zyklischen oder vorzugsweise linearen C₁₋₂₀-Alkyl-, -Alkenyl-, -Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, zyklischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰-unterbrochen und durch eine oder mehrere polymerisationsfähige Gruppen und/oder Reste R⁹ substituiert sein kann;
- R⁴, R⁵: unabhängig voneinander jeweils H, Halogen, ein verzweigter oder linearer C₁₋₆-Alkyl- oder -O-C₁₋₆-Alkylrest sind;
- R⁶, R⁷, R⁸: unabhängig voneinander jeweils H, Halogen, ein verzweigter, zyklischer oder vorzugsweise linearer C₁₋₂₀-Alkyl-, -Alkenyl-, -Alkyloxy- oder -Alkenoxy-Rest sind, der ein- oder mehrfach durch O, S oder -NR²⁰- unterbrochen und durch eine oder mehrere polymerisationsfähige Gruppen und/oder Reste R⁹ substituiert sein kann;
- R⁹: -OH, -CₓF₂ₓ₊₁ mit x = 1 bis 20, -[Si(CH₃)₂]_{y}-CH₃ mit y = 1 bis 20 ist; und
- R²⁰: H, Halogen, ein verzweigter, zyklischer oder vorzugsweise linearer C₁₋₂₀-Alkyl-, -Alkenyl-, -Alkyloxy- oder -Alkenoxy-Rest ist.

Durch die Formel (I) und die übrigen hierin gezeigten Formeln werden sämtliche stereoisomere Formen sowie Gemische verschiedener stereoisomerer Formen, wie z.B. Racemate, erfaßt. Die Formeln erfassen nur solche Verbindungen, die mit der chemischen Valenzlehre vereinbar sind.

Der Hinweis, daß eine Rest durch O unterbrochen sein kann, ist so zu verstehen, daß die O-Atome in die Kohlenstoffkette des Restes eingeschoben werden, d.h. beidseitig von Kohlenstoffatomen begrenzt werden. Die Anzahl der O-Atome ist daher um mindestens 1 kleiner als die Zahl der Kohlenstoffatome, und die O-Atome können nicht endständig sein. Erfindungsgemäß sind Reste, die nicht durch O-Atome unterbrochen sind, bevorzugt.

Halogen (abgekürzt Hal) steht vorzugsweise für F, Cl, Br oder I, insbesondere F, Cl, ganz besonders bevorzugt Cl.

Bevorzugte polymerisationsfähige Gruppen, die bei den obigen Resten als Substituenten vorhanden sein können, sind Vinyl, Styryl, (Meth)acrylat, (Meth)acrylamid und/oder N-Alkylacrylamid, besonders bevorzugt (Meth)acrylat, (Meth)acrylamid und/oder N-Alkylacrylamid. Vorzugsweise sind die Reste R², R³, R⁶, R⁷ und R⁸ mit 0 bis 3, insbesondere 0 bis 1 polymerisierbaren Gruppen substituiert. Die polymerisationsfähigen Gruppen sind vorzugsweise endständig angeordnet.

Erfindungsgemäß sind solche Verbindungen der allgemeinen Formeln (I) und (II) bevorzugt, bei denen die Variablen die folgenden Bedeutungen haben, die unabhängig voneinander gewählt werden können:
- R¹: 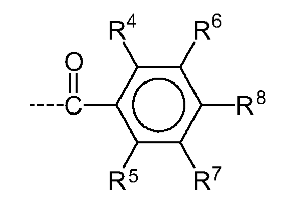 oder H, oder eine der für R² und R³ angegebenen Bedeutungen;
- R², R³: unabhängig voneinander ein linearer C₁₋₄-Alkyl-, oder -Alkenyl-Rest, der durch ein oder mehrere polymerisationsfähigen Gruppen substituiert sein kann;
- R⁴, R⁵: unabhängig voneinander jeweils H, Halogen, ein verzweigter oder linearer C₁₋₄-Alkyl- oder -O-C₁₋₄-Alkylrest;
- R⁶, R⁷, R⁸: unabhängig voneinander jeweils H, Halogen, ein linearer C₁₋₂₀-Alkyl-, -Alkenyl-, -Alkoxy- oder -Al-kenoxy-Rest, der durch ein oder mehrere -O-, -S-oder -NR²⁰- unterbrochen und durch ein oder mehrere polymerisationsfähige Gruppen substituiert sein kann.

Besonders bevorzugte Definitionen der Variablen, die ebenfalls unabhängig voneinander gewählt werden können, sind:
- R¹: 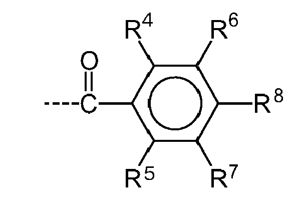 oder eine der für R² und R³ angegebenen Bedeutungen;
- R², R³: C₁-C₄-Alkyl;
- R⁴, R⁵, R⁸: H, Cl, CH₃, OCH₃;
- R⁶,R⁷: H, C₁-C₄-Alkyl, das durch ein oder mehrere O-Atome unterbrochen sein kann.

Ganz besonders bevorzugt sind Verbindungen der Formel (II) in denen R²=R³, R⁴=R⁵ und/oder R⁶=R⁷.

Besonders bevorzugt sind naturgemäß solche Verbindungen, bei denen alle Variablen eine der bevorzugten und insbesondere der besonders bevorzugten Bedeutungen haben.

Es sind solche Acylgermane gemäß der Formel (I) und insbesondere der Formel (II) bevorzugt, die 0 bis 2, vorzugsweise 0 oder 1 polymerisationsfähige Gruppe enthalten. Die einzelnen Reste der Formel (I) enthalten vorzugsweise 0 bis 4, besonders bevorzugt 0 bis 2 polymerisierbare Gruppen.

Konkrete Beispiele für besonders bevorzugte Verbindungen sind:

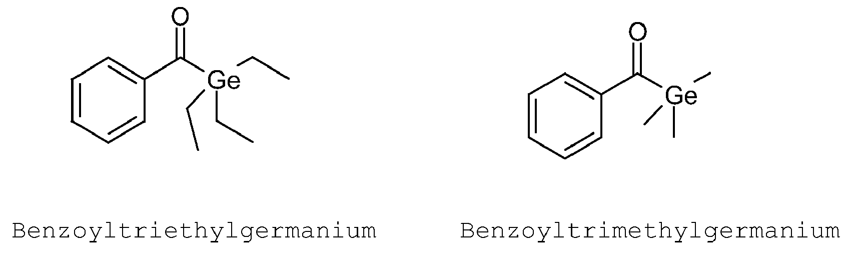

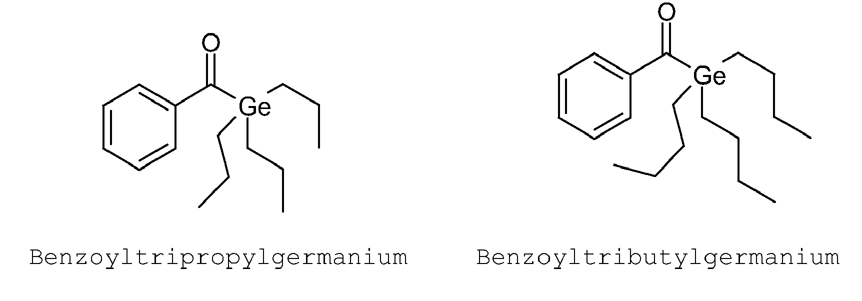

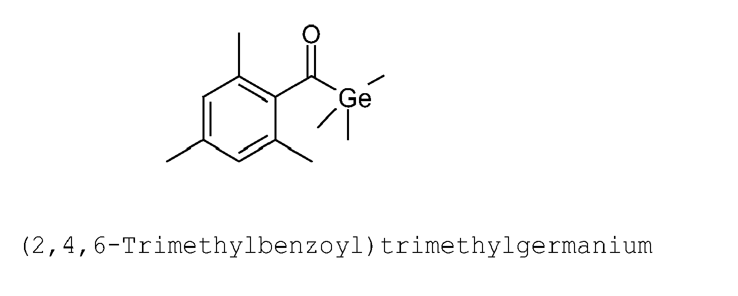

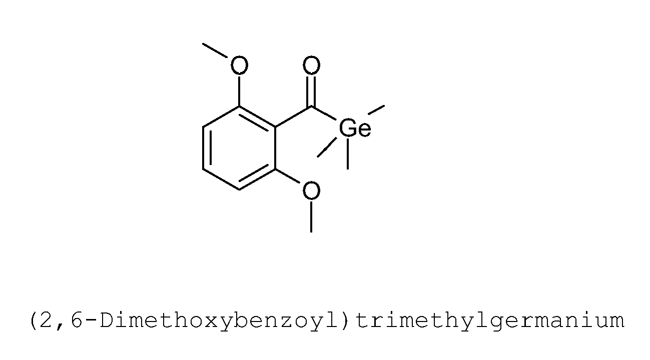

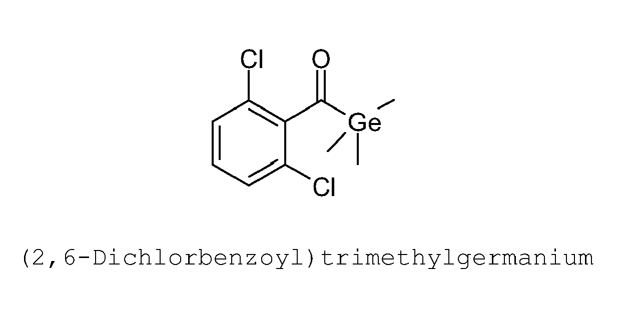

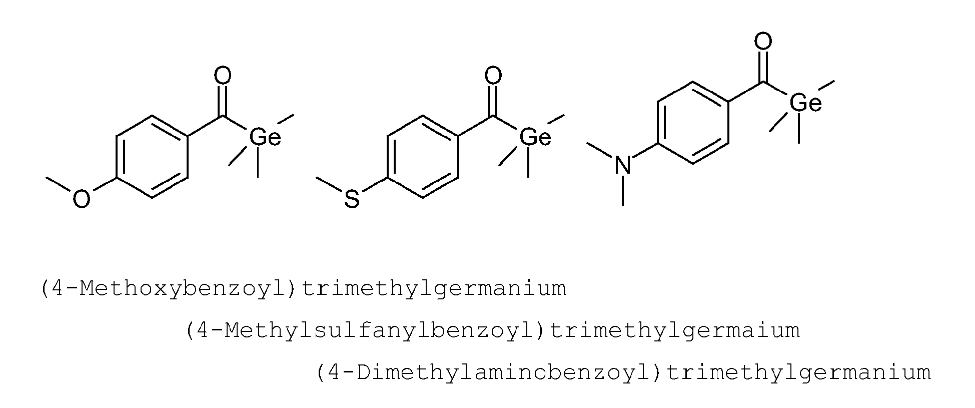

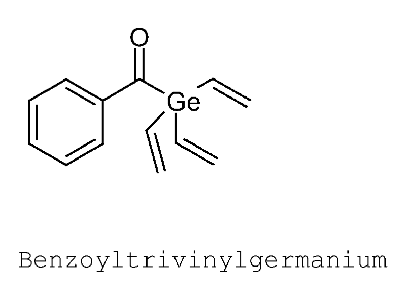

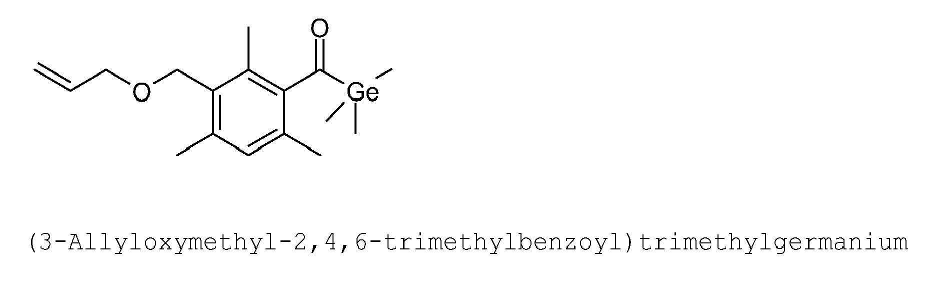

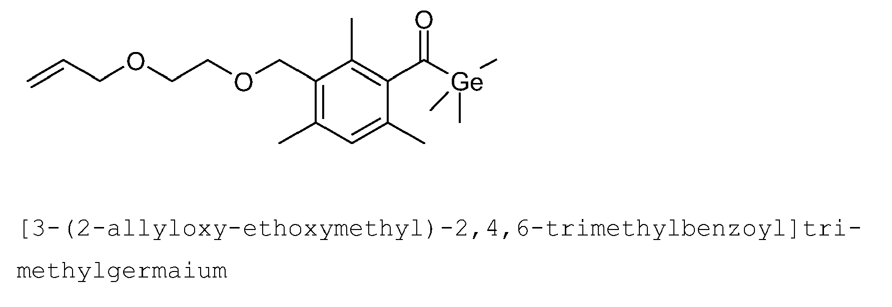

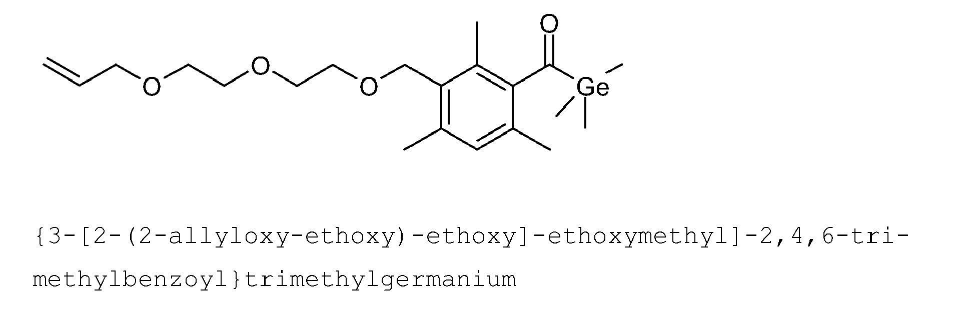

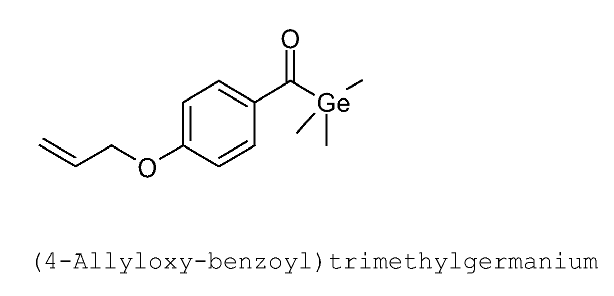

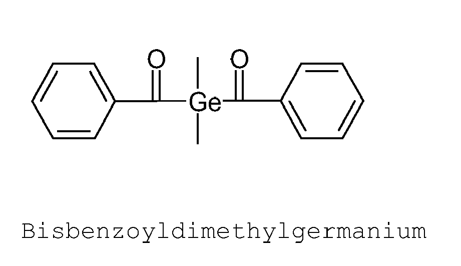

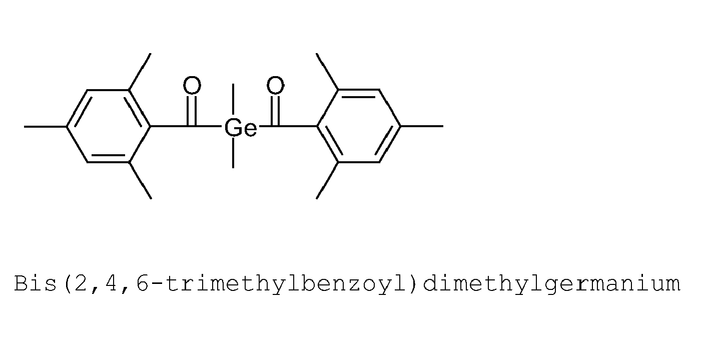

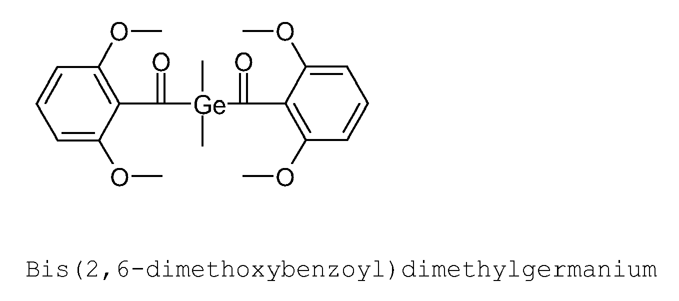

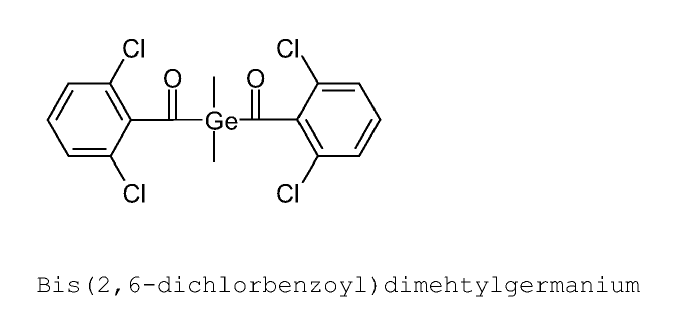

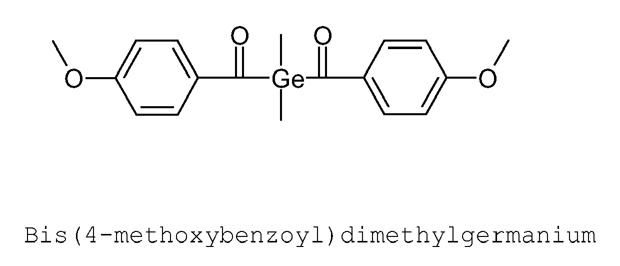

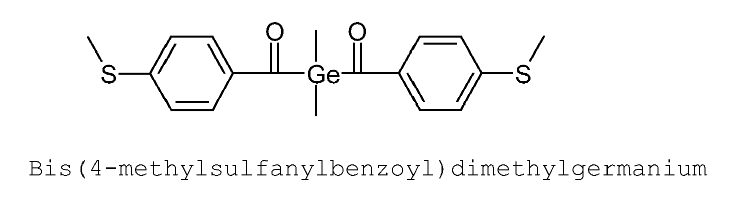

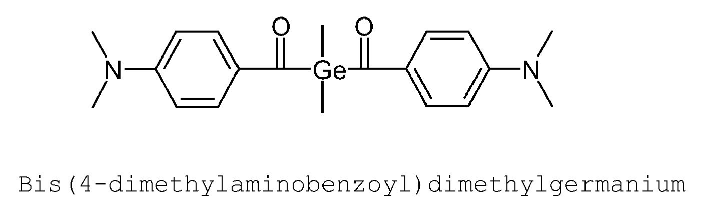

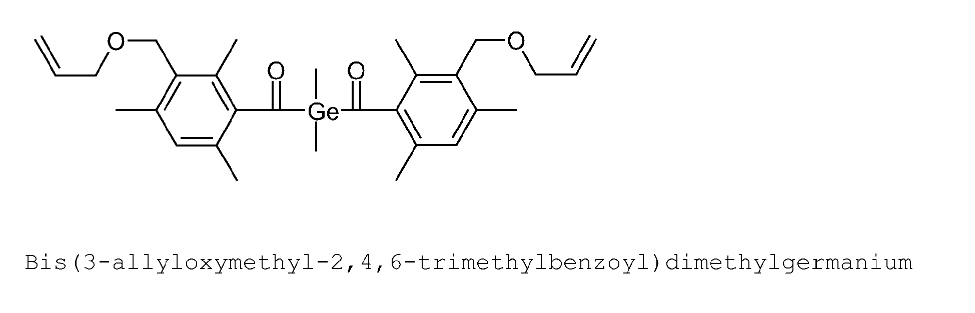

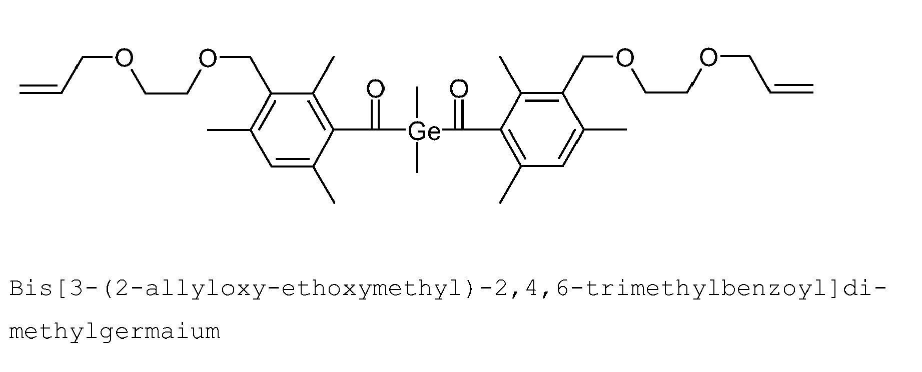

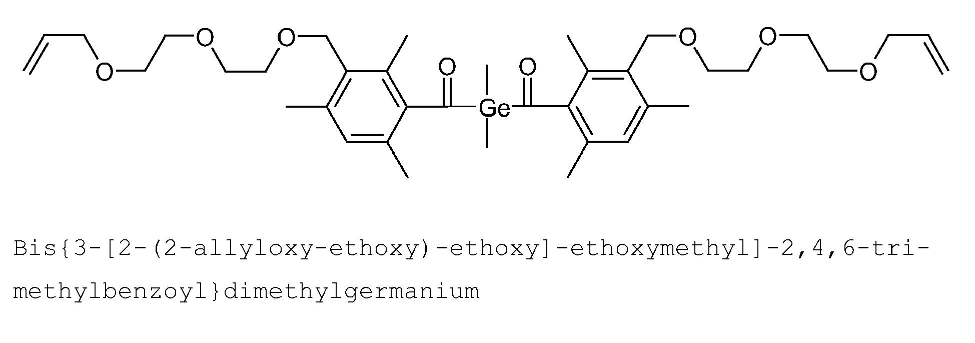

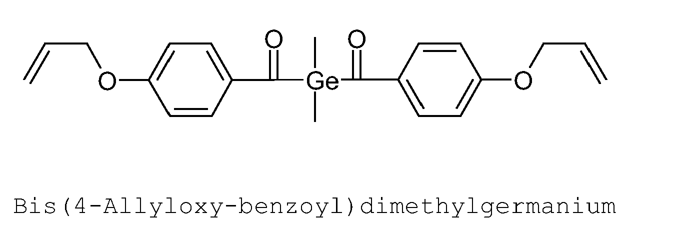

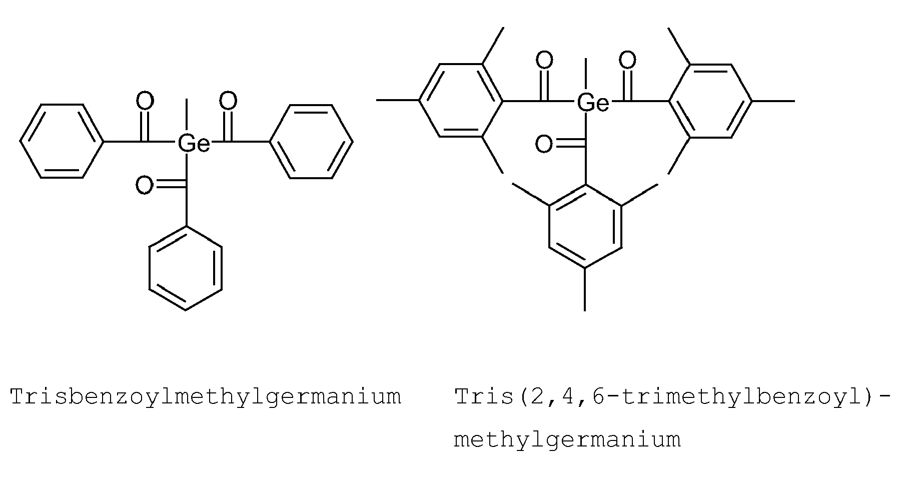

Ebenso bevorzugt sind die zu den oben gezeigten Verbindungen strukturähnlichen Verbindungen (2,4,6-Trimethylbenzoyl)triethylgermanium, (2,4,6-Trimethylbenzoyl)tripropyl-germanium, (2,4,6-Trimethylbenzoyl)tributylgermanium, (2,6-Dimethoxybenzoyl)triethylgermanium, (2,6-Dimethoxybenzoyl)-tripropylgermanium, (2,6-Dimethoxybenzoyl)tributylgermanium, (2,6-Dichlorbenzoyl)triethylgermanium, (2,6-Dichlorbenzoyl)tripropylgermanium, (2,6-Dichlorbenzoyl)tributylgermanium, Bisbenzoyldiethylgermanium, Bisbenzoyldipropylgermanium, Bisbenzoyldibutylgermanium, Bis(2,4,6-trimethylbenzoyl)diethylgermanium, Bis(2,4,6-trimethylbenzoyl)-diproplgermanium, Bis(2,4,6-trimethylbenzoyl)dibutylgermanium, Bis(2,6-dimethoxybenzoyl)diethylgermanium, Bis(2,6-dimethoxybenzoyl)dipropylgermanium, Bis(2,6-dimethoxybenzoyl)dibutylgermanium, Bis(2,6-dichlorbenzoyl)-benzoyl)dibutylgermanium, Bis(2,6-dichlorbenzoyl)diehtylgermanium, Bis(2,6-dichlorbenzoyl)dipropylgermanium, Bis(2,6-dichlorbenzoyl)dibutylgermanium, Trisbenzoylethylgermanium und Tris(2,4,6-trimethylbenzoyl)ethylgermanium.

Die erfindungsgemäß verwendeten Acylgermane der allgemeinen Formel (I) sind zum Teil bereits aus dem Stand der Technik bekannt. Die Synthese der Monoacylgermane kann z.B. nach einer Methode von Yamamoto et. al. (Yamamoto, K.; Hayashi, A.; Suzuki, S.; Tsuji J.; Organometallics; 6 (1987) 974) durch Umsetzung von Hexaalkyldigermanium mit Säurechlorid erfolgen:

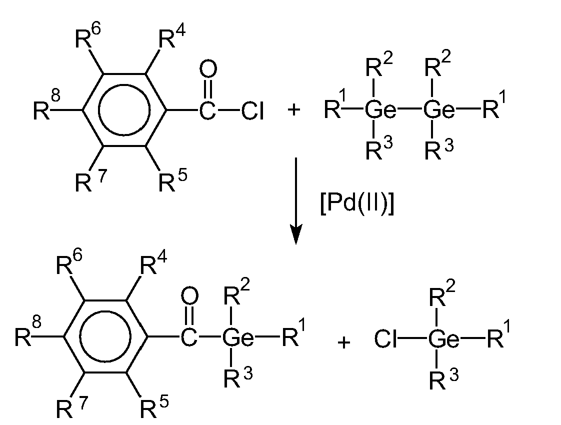

### Konkretes Beispiel:

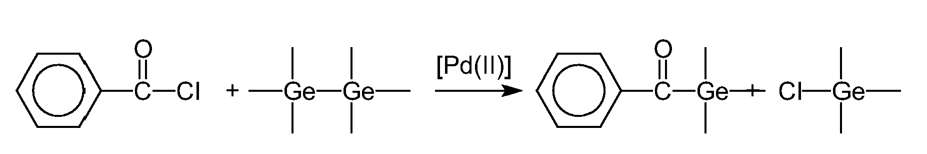

Eine Möglichkeit Bisacylgermane herzustellen, ist die Umsetzung der entsprechenden lithierten Germaniumverbindungen mit Säurechloriden nach Castel et. al. (Castel, A.; Riviere, P.; Satgé, J.; Ko, H.Y.; Organometallics; 9 (1990) 205):

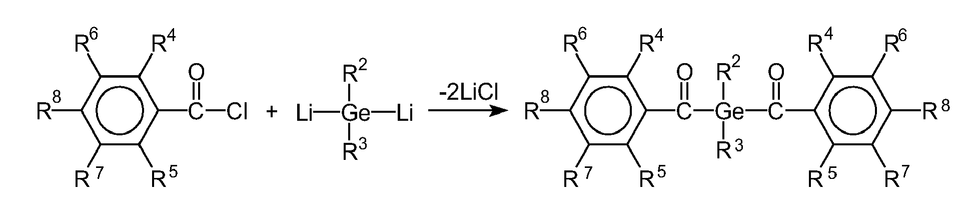

### Konkretes Beispiel:

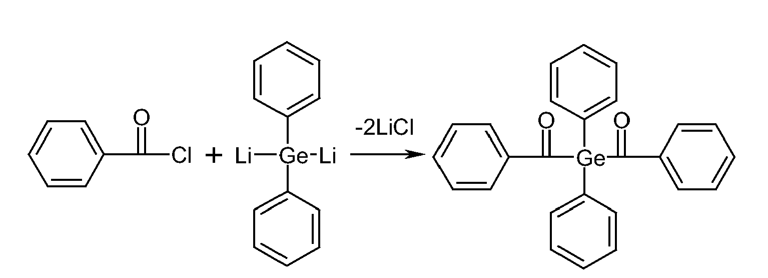

Die Herstellung von lithierten aromatischen Germaniumverbindungen kann z.B. durch Reaktion des entsprechenden Germaniumhalids (X = Halogen) mit Lithium (Li) (Nishimura, T.; Inoue-Ando, S.; Sato, Y., J. Chem. Soc., Perkin Trans.1; (1994) 1589) oder des Hydrogermaniums mit n-Butyllithium (BuLi) erfolgen (Castel, A.; Riviere, P.; Satgé, J.; Ko, H.Y.; Organometallics; 9 (1990) 205):

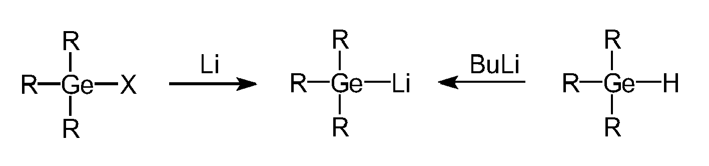

Des weiteren lassen sich Mono- und Bisacylgermane synthetisieren, indem ein Carbanion, welches aus 1,3-Dithianen erhalten wird, mit Germaniumchloriden nach Brook et. al. umgesetzt wird (Brook, A. G.; Duff, J. M.; Jones, P. F.; Davis, N. R.; "Synthesis of Silyl and Germyl Ketones" J. Am Chem. Soc. 89(2), 431 - 434 (1967)). Dieser Syntheseweg eignet sich besonders zur Herstellung von Bisalkylbisacylgermanen:

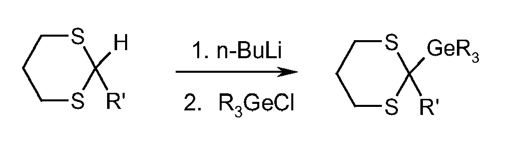

Die erhaltenen Dithiane können nach Methoden, die dem Fachmann allgemein bekannt sind, zu den entsprechenden Ketonen hydrolysiert werden (nach Brook, A. G.; Duff, J. M.; Jones, P. F.; Davis, N. R.; "Synthesis of Silyl and Germyl Ketones" J. Am Chem. Soc. 89(2), 431 - 434 (1967) oder z.B. auch nach Sharma, H.K.; Cervanes-Lee, F.; Pannel, K. H.; "Organometalloidal derivatives of the transition metals, XXVII. Chemical and structural investigations on (ferrocenylacyl)germanes):

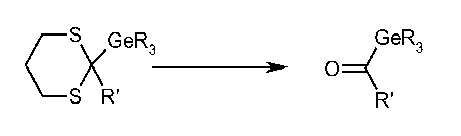

Die Acylgermane der allgemeinen Formel (I) eignen sich besonders als Photoinitiatoren für die Polymerisation, insbesondere als Initiatoren für die radikalische Polymerisation, Photoaddition und für die Thiol-En-Reaktion (Polyaddition). Es wurde gefunden, daß mit diesen Initiatoren bei Bestrahlung mit Licht, vorzugsweise im sichtbaren Bereich, insbesondere mit einer Wellenlänge von 400 bis 500 nm, im Vergleich zu herkömmlichen Photoinitiatoren eine hohe Durchhärtungstiefe erzielt werden kann, ohne daß die Initiatoren zu gefärbten Materialien führen. Die ist bei vielen technischen und besonders medizinischen Werkstoffen, wie z.B. Dentalwerkstoffen und Knochenzementen von großem Vorteil.

Darüber hinaus zeichnen sich die erfindungsgemäß verwendeten Acylgermane der Formel (I) im Vergleich zu herkömmlichen Initiatoren durch eine geringe Cytotoxizität aus, was für medizinische Anwendungen ebenfalls ein besonderer Vorteil ist. Die Acylgermane eignen sich daher beispielweise auch als Initiatoren für Materialien zur Herstellung von Kontaklinsen aber auch von gewöhnlichen optischen Linsen, die von der geringen Verfärbungsneigung der Initiatoren profitieren.

Die Verwendung der Initiatoren der Formel (I) ist nicht auf medizinische Anwendungen beschränkt. Die große Durchhärtungstiefe bei der Härtung mit Licht im sichtbaren Wellenlängenbereich ist auch bei technischen Anwendungen ein erheblicher Vorteil. Die erfindungsgemäßen Zusammensetzungen eignen sich für eine Vielzahl von Anwendungszwecken, wie zum Beispiel als Druck- oder Anstrichfarben, Lacke, Adhäsive, zur Herstellung von Druckplatten, integrierten Schaltkreisen, Photoresists, Lötmasken, Tinten für Farbdrucker, als Materialien für die holographische Datenspeicherung, zur Herstellung von nano-dimensionierten mikroelektromechanischen Elementen, Lichtwellenleitern, Formteilen und zur optischen Herstellung von Informationsträgern.

Zur Initiierung der Polymerisation werden die Acylgermane der Formel (I) vorzugsweise mit Licht im Wellenlängenbereich von 200 bis 750 nm, besonders bevorzugt 200 bis 550 nm, weiter bevorzugt 300 bis 550 nm und ganz besonders bevorzugt 350 bis 500 nm bestrahlt. Sie sind damit als Initiatoren für die Laser-Härtung und die Laser induzierten 3D Härtung sowie für die 2-Photonen Polymerisation brauchbar. Sie eignen sich insbesondere als Initiatoren für pigmentierte Systeme, da sie die Nutzung von Absorptionslücken des Pigments ermöglichen.

Besonders vorteilhaft ist, daß die Initiatoren auch mit LED Lichtquellen aktiviert werden können. Die Wellenlänge von LED's ist abhängig von der Gitterkonstante des Substrates. Die Qualität (thermische Festigkeit, Wärmeausdehnung, Konstanz der Atomabstände etc.) des Substrats bestimmt die Höhe der möglichen Leistung der LED's. In der intra-oralen Anwendung sind Wellenlängen erst ab etwa 380 nm erlaubt, so daß Initiatoren der Formel (I), die sich mit einer Wellenlänge im Bereich von 380 nm und mehr aktivieren lassen, besonders bevorzugt sind.

Gegenstand der Erfindung sind auch Kombinationen von LED-Lichtquellen mit Initiatoren gemäß der Formel (I) oder mit Zusammensetzungen, die einen solchen Initiator enthalten. Zur dentalen Anwendung sind Systeme von LED-Lichtquellen mit einer Wellenlänge im Bereich von 400 bis 550 nm, vorzugsweise 400 bis 480 nm und insbesondere 450 ± 20 nm, und darauf abgestimmten Initiatoren bzw. Zusammensetzungen bevorzugt, d.h. Initiatoren mit einer Aktivierungswellenlänge im Bereich von 400 bis 550 nm, vorzugsweise 400 bis 480 nm und ganz besonders bevorzugt ca. 450 ± 20 nm und diese enthaltende Zusammensetzungen. Daneben sind LED-Lichtquellen mit einer Wellenlänge von etwa 650 ± 30 nm oder etwa 360 ± 30 nm zusammen mit darauf abgestimmten Initiatoren oder Zusammensetzungen erfindungsgemäß bevorzugt.

Die erfindungsgemäßen Zusammensetzungen enthalten neben mindestens einem Acylgerman der Formel (I) vorzugsweise auch ein polymerisierbares Bindemittel. Bevorzugt sind Bindemittel auf der Basis radikalisch polymerisierbarer Monomere und/oder Prepolymere.

Als radikalisch polymerisierbare Bindemittel eignen sich besonders mono- oder multifunktionelle (Meth)acrylate oder deren Mischung. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden.

Diesbezügliche Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)-acrylat, Trimethylolpropantri(meth)acrylat, Pentaerythrittetra(meth)acrylat, sowie Glycerindimethacrylat, 1,4-Butandioldi(meth)acrylat, 1,10-Decandioldi(meth)acrylat oder 1,12-Dodecandioldi(meth)acrylat. Zusammensetzungen, die mindestens ein radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Als radikalisch polymerisierbare Bindemittel lassen sich auch hydrolysestabile Monomere, wie hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid, N-Methyl-N-(2-hydroxyethyl)-acrylamid oder N-monosubstituierte Methacrylamide, wie z.B. N-Ethylmethacrylamid oder N-(2-Hydroxyethyl)methacrylamid sowie außerdem N-Vinylpyrrolidon oder Allylether einsetzen. Bevorzugte Beispiele für hydrolysestabile Vernetzermonomere sind Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechorid synthetisiert werden können. Bevorzugt sind bei Raumtemperatur flüssige Monomere, die als Verdünnermonomere eingesetzt werden können.

Weiterhin lassen sich als radikalisch polymerisierbare Bindemittel auch schrumpfungsarme radikalisch ringöffnend polymerisierbare Monomere wie z.B. von mono- oder multifunktionelle Vinylcyclopropane oder bicyclische Cyclopropanderivate, vorzugsweise die in DE 196 16 183 C2 oder EP 1 413 569 beschriebenen, oder cyclische Allylsulfide, vorzugsweise die in US 6,043,361 und US 6,344,556 beschriebenen, einsetzen. Diese können auch in Kombination mit den voranstehend aufgeführten Di(meth)acrylat-Vernetzern verwendet werden. Bevorzugte ringöffnend polymerisierbare Monomere sind Vinylcyclopropane, wie 1,1-Di(ethoxycarbonyl)- oder 1,1-Di(methoxycarbonyl)-2-vinylcyclopropan, die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester, deren Disubstitutionsprodukte in 3-Stellung wie (3,3-Bis(ethoxycarbonyl)bicyclo[3.1.0]hex-1-yl)acrylsäuremethyl-oder -ethylester. Bevorzugte cyclische Allylsulfide sind die Additionprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren (Desmodur^{®} VP LS 2294 der Bayer AG).

Außerdem können als radikalisch polymerisierbare Bindemittel auch Styrol, Styrolderivate oder Divinylbenzol, ungesättigte Polyester-, Polyurethan- und Epoxy-Harze sowie Allylverbindungen oder radikalisch polymerisierbare Polysiloxane, die aus geeigneten Methacrylsilanen, wie z.B. 3-(Methacry-loyloxy)propyltrimethoxysilan, hergestellt werden können und z.B. in der DE 199 03 177 C2 beschrieben sind, eingesetzt werden.

Weiterhin lassen sich als radikalisch polymerisierbare Bindemittel auch Mischungen der voran stehend genannten Monomeren mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyl-trimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich auch radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritol-pentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)-hexyldihydrogenphosphat und 1,3-Bis-(N-acryloyl-N-propyl-amino)-propan-2-yl-dihydrogenphosphat.

Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Als durch Polyaddition härtbare Bindemittel eignen sich besonders Thiol-En-Harze, die Mischungen von mono- oder multifunktionellen Mercaptoverbindungen und di- oder multifunktionellen ungesättigten Monomeren, vor allem Allyl- oder Norbonenverbindungen enthalten.

Beispiele für mono- oder multifunktionelle Mecaptoverbindungen sind o-, m- oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit.

Beispiele für di- oder multifunktionelle Allylverbindungen sind Ester von Allylalkohol mit Di- oder Tricarbonsäuren, wie Malon- Malein- Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat.

Beispiele für di- oder multifunktionelle Norbonenverbindungen sind Diels-Alder-Additionsprodukte von Cyclopentadien oder Furan mit di- oder multifunktionellen (Meth)acrylaten, sowie Ester und Urethane von 5-Norbornen-2-methanol oder 5-Norbornen-2-ol mit Di- oder Polycarbonsäuren, wie z.B. Malon-Malein- Glutar-, Bernstein-, Adipin-, Sebacin-, Phthal-, Terephthal- oder Gallussäure, mit Di- oder Polyisocyanaten, wie Hexamethylendiisocyanat oder dessen cyclischem Trimer, 2,2,4-Trimethylhexamethylendiisocyanat, Toluylendiisocyanat oder Isophorondiisocyanat.

Neben Acylgerman der allgemeinen Formel (I) können die erfindungsgemäßen Zusammensetzungen zusätzlich auch bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acyl- oder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon sowie ggf. Coinitiatoren (Welcher Begriff ist hier richtig?) wie. tertiäre Amine z.B Dimethylaminobenzoesäureethylester oder Methyldiethanolamin enthalten.

Weiterhin können die erfindungsgemäßen Zusammensetzungen neben den Acylgermanen der allgemeinen Formel (I) zur dualen Härtung auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid enthalten. Zur Beschleunigung der Initiierung mittels Peroxiden lassen sich Kombinationen mit aromatischen Aminen einsetzen. Bevorzugte Redoxsysteme sind Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und Reduktionsmitteln, wie z.B. Ascorbinsäure, Barbiturate oder Sulfinsäuren, für die duale Aushärtung geeignet. Die Menge an zusätzlichen Initiatoren liegt vorzugsweise im Bereich von 0 bis 3 Gew.-%. Ein Vorteil der erfindungsgemäßen Initiatoren der Formel (I) ist, daß sie keine Coinitiatoren oder Aktivatoren zur Beschleunigung erfordern und ohne solche eingesetzt werden können.

Erfindungsgemäß sind Zusammensetzungen bevorzugt, die einen oder mehrere Füllstoffe, vorzugsweise organische oder anorganische partikuläre Füllstoffe enthalten. Bevorzugte anorganische partikulären Füllstoffe sind amorphe kugelförmige nanopartikuläre Füllstoffe auf der Basis von Oxiden, wie pyrogener Kieselsäure oder Fällungskieselsäure, ZrO₂ und TiO₂ oder Mischoxiden von SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid, nanopartikuläres Tantal(V)-oxid oder Bariumsulfat. Darüber hinaus können auch faserförmige Füllstoffe wie Nanofasern, Glasfasern, Polyamid-oder Kohlenstoff-Fasern eingesetzt werden.

Als weite Komponente können die erfindungsgemäßen Zusammensetzungen Farbmittel wie Farbstoffe und/oder Pigmente enthalten.

Außerdem können die erfindungsgemäßen Zusammensetzungen im Bedarfsfalle weitere Additive sowie Lösungsmittel, wie z.B. Wasser, Ethanol, Aceton und/oder Ethylacetat, enthalten.

Die Additive sind vorzugsweise aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufheller, Fließverbesserern, Verdikkern und Antischaummitteln ausgewählt.

Die Initiatoren gemäß den Formeln (I) und (II) zeichnen sich durch eine hohe Reaktivität aus und können daher in geringen Konzentrationen eingesetzt werden (vgl. Beispiel 7). Die erfindungsgemäßen Zusammensetzung enthalten, bezogen auf die Gesamtmasse der Zusammensetzung, vorzugsweise 0,001 bis 5 Gew.-%, besonders bevorzugt 0,01 bis 4 Gew.-% und insbesondere 0,1 bis 3 Gew.-% Acylgerman der Formel (I).

Die erfindungsgemäßen Materialien enthalten somit vorzugsweise:
(a) 0,001 bis 5 Gew.-% Acylgerman der allgemeinen Formel (I), vorzugsweise 0,01 bis 4 Gew.-%, besonders bevorzugt 0,1 bis 3 Gew.-%,
(b) 5 bis 99,9 Gew.-% polymerisierbares Bindemittel, vorzugsweise 10 bis 95 Gew.-%, besonders bevorzugt 15 bis 90 Gew.-%, und
(c) 0 bis 90 Gew.-% Füllstoff, vorzugsweise 5 bis 87 Gew.-%, besonders bevorzugt 10 bis 85 Gew.-%.

Die Zusammensetzungen können vorteilhaft außerdem enthalten:
(d) 0 bis 50 Gew.-% Additiv, vorzugsweise 0,01 bis 4 Gew.-%, besonders bevorzugt 0,1 bis 3 Gew.-%, wobei sich diese Mengenangaben auf die Gesamtmasse aller Additive beziehen, und
(e) 0 bis 10 Gew.-%, vorzugsweise 0.01 bis 5 Gew.-% Pigmente und/oder Farbstoffe.

Alle Prozentangaben beziehen sich, wenn nicht anders angegeben, auf die Gesamtmasse der Zusammensetzung.

Die erfindungsgemäßen Zusammensetzungen eignen sich besonders als Adhäsive, Beschichtungen, Lacke, Tinten, Zemente, Komposite, zur Herstellung von Formteilen bzw. Formkörpern, wie Stäben, Platten, Scheiben, optischen Linsen, Kontaktlinsen, und insbesondere als Dentalwerkstoffe, ganz besonders als Füllungskomoposite.

Zusammensetzungen zur Anwendung als dentale Zemente enthalten bevorzugt:
(a) 0,001 bis 3 Gew.-% Acylgerman der allgemeinen Formel (I),
(b) 20 bis 70 Gew.-% polymerisierbares Bindemittel,
(c) 30 bis 75 Gew.-% Füllstoff und
(d) 0,01 bis 5 Gew.-% Additiv.

Zusammensetzungen zur Anwendung als dentale Komposite enthalten bevorzugt:
(a) 0,001 bis 2 Gew.-% Acylgerman der allgemeinen Formel (I),
(b) 10 bis 60 Gew.-% polymerisierbares Bindemittel,
(c) 40 bis 85 Gew.-% Füllstoff und
(d) 0,01 bis 5 Gew.-% Additiv.

Zusammensetzungen zur Anwendung als dentale Beschichtungsmaterialien enthalten bevorzugt:
(a) 0,001 bis 5 Gew.-% Acylgerman der allgemeinen Formel (I),
(b) 20 bis 99,9 Gew.-% polymerisierbares Bindemittel,
(c) 0 bis 20 Gew.-% nanopartikuläre Füllstoffe und
(d) 0,01 bis 2 Gew.-% Additiv,
(e) 0 bis 50 Gew.-% Lösungsmittel.

Zusammensetzungen zur Anwendung als Druckertinte enthalten bevorzugt:
(a) 0,001 bis 5 Gew.-% Acylgerman der allgemeinen Formel (I),
(b) 30 bis 60 Gew.-% polymerisierbares Bindemittel,
(c) 1 bis 45 Gew.-% Farbmittel und
(d) 0,01 bis 30 Gew.-% Additiv.

Zusammensetzungen zur Anwendung als Lack, beispielsweise als Weißlack oder als Lack für optische Fasern, enthalten bevorzugt:
(a) 0,001 bis 5 Gew.-% Acylgerman der allgemeinen Formel (I),
(b) 55 bis 99,5 Gew.-% polymerisierbares Bindemittel,
(c) 0,1 bis 50 Gew.-% Pigment.

Ein bevorzugtes Pigment zur Herstellung von Lacken ist Ti0₂.

Dentalwerkstoffe, die sich durch Thiol-en-Reaktion härten lassen, enthalten vorzugsweise eine Mischung aus einer oder mehreren Polythiolverbindungen und einer oder mehreren Polyvinylverbindungen, wobei eine oder mehrere dieser Verbindungen in oligomerer Form vorliegen können. Vorzugsweise sind 45 bis 55 % der funktionellen Gruppen dieser Mischungen Thiolgruppen, die übrigen Gruppen können Vinylgruppen sein. Die Mischungen können weiterhin einen oder mehrere Füllstoffe enthalten, wobei der Menge an polymerisierbaren Harzen vorzugsweise im Bereich von 10 bis 40 Gew.-% und die Füllstoffmenge vorzugsweise im Bereich von 60 bis 90 Gew.-% liegt. Geeignete Mischungen aus Polythiol- und Polyvinylverbindungen sowie geeignete füllstoffhaltige Mischungen werden in der WO 2005/086911 beschrieben. Die Menge an Initiator gemäß Formel (I) beträgt vorzugsweise 0,05 bis 0,5 Gew.-%.

Gegenstand der Erfindung ist auch die Verwendung von Acylgermanen der Formel (I) zur Herstellung von Adhäsiven, Beschichtungen, Lacken, Tinten, Zementen, Kompositen, Formteilen oder Dentalwerkstoffen sowie deren Verwendung als Initiator für die radikalische Polymerisation.

Außerdem betrifft die Erfindung ein Verfahren zur Herstellung von Formkörpern, insbesondere dentalen Kronen, Brücken, Inlays und künstlichen Zähnen, bei dem man eine erfindungsgemäße Zusammensetzung auf an sich bekannte Weise zu dem Formkörper formt und dann zumindest teilweise, vorzugsweise vollständig, härtet. Die Härtung erfolgt vorzugsweise durch radikalische Polymerisation.

Die erfindungsgemäßen Photoinitiatoren zeichnen sich besonders durch eine hohe Reaktivität und eine hohe Aktivität schon bei geringer Einsatzkonzentration aus. Dadurch kann einer äußerst rasche Aushärtung des Photopolymers im Vergleich zu bekannten Photoinitiatoren, die im sichtbaren Bereich absorbieren erreicht werden. Beispielsweise ergaben Messungen von Bisacyldiethylgermanium in einer Harzmischung aus Decandioldimethacrylat (D₃MA):UDMA:Bis-GMA = 1:1:1 eine beinahe doppelt so hohe Polymerisationsrate (Rp) wie Campherchinon in Kombination mit einem Aminbeschleuniger in der gleichen Formulierung. Die Aushärtungszeit konnte im Vergleich zu Campherquinon/Amin ebenfalls halbiert werden. Selbst bei 15-facher Verdünnung an Bisacyldiethylgermanium kann noch immer eine mit Campherchinon/Amin als Photoinitiator vergleichbare Reaktivität erreicht werden (siehe Ausführungsbeispiele, Tabellen 7, 8, 9, Summe aus Initiator und Beschleuniger).

Außerdem weisen die naturgemäß gelblich gefärbten Photoinitiatoren gemäß Formel (I) einen ausgezeichneten Photobleachingeffekt auf, d.h. die Verbindungen der Formel (I) werden bei der Härtung entfärbt und dadurch Verfärbungen des Material nach dem Aushärten vermieden (siehe Ausführungsbeispiele, Tabelle 2).

Die Erfindung wird im Folgenden anhand von Beispielen näher erläutert.

### Ausführungsbeispiele

### Beispiel 1: Synthese von Benzoyltrimethylgermanium

In einem trockenen 50 ml Dreihalskolben mit Rückflusskühler und Septum wurden unter Argon 1.64 g (4.49 mmol) Allylpalladium(II)chlorid Dimer, 1.49 g (8.97 mmol) Triethylphosphit und 23.24 g (98.7 mmol) Hexamethyldigermanium vorgelegt, und 5 min bei Raumtemperatur gerührt. Anschließend wurde 12.62 g (89.7 mmol) frisch destilliertes Benzoylchlorid zugetropft. Nach 4h Rühren bei 110°C, wurde der Pd-Katalysator vom Reaktionsgemisch abgetrennt und flüchtige Reaktionsprodukte sowie der Überschuß an Hexamethyldigermanium am Rotationsverdampfer abgezogen. Das Reaktionsgemisch wurde säulenchromatographisch (Petrolether (PE) : Ethylacetat (EE) = 40:1) aufgetrennt. Es ergaben sich 7.8 g (78 % d. Th.) an Benzoyltrimethylgermanium als gelbe Flüssigkeit. DC (Petrolether: Ethylacetat = 20:1): R_{f}=0.58.
UV-VIS: λₘₐₓ: 411,5 nm, ε = 1374 dm²/mol
¹H-NMR (200 MHz; CDCl₃): δ (ppm): 7.78-7.82 (m, 2H, Ar-H^{2,6}), 7.48-7.58 (m, 3H, Ar-H^{3,4,5}), 0.51 (s, 9H, -CH₃).
¹³C-NMR (200 MHz; CDCl₃): δ (ppm): 234.39 (-C=O), 140.61 (Ar-C¹), 132.90 (Ar-C⁴), 128.75 (Ar-C^{2,6}), 127.71 (Ar-C^{3,5}), -1.14 (-CH₃).
IR (cm⁻¹): 2979, 2916, 1628 (C=O), 1582, 1448, 1310, 1239, 1207, 1172, 905, 827, 770, 732.

### Beispiel 2: Synthese von Diethylbis(2-phenyl-1,3-dithian-2-yl) germanium

In einem trockenen 50 ml Dreihalskolben wurden unter Argon 1.85 g (9.42 mmol) 2-Phenyl-1,3-dithian vorgelegt, und in 28 ml wasserfreiem THF gelöst. Bei 0 °C wurden 3.99 ml 2.36 M BuLi-Lösung in Hexan zugetropft und die Reaktionslösung 2 h bei 0 °C gerührt. 0.83 mg (3.93 mmol) Diethyldichlorgermanium gelöst in 8 ml wasserfreiem THF wurden langsam bei 0°C zum Reaktionsgemisch getropft und anschließend weitere 2 h bei O °C gerührt. Zur Vervollständigung der Reaktion wurde eine weitere Lösung von 2-Phenyl-2-lithium-1,3-dithian (2.36 mmol) wie zuvor beschrieben hergestellt und bei 0 °C zur Reaktionslösung getropft, welche anschließend 18 h bei 6°C gerührt wurde. Die Reaktion wurde durch Zugabe von 20 ml Wasser gequencht und das Rohprodukt mit Diethylether (3 x 30 ml) extrahiert. Die vereinigten organischen Phasen wurden mit Na₂SO₄ getrocknet und das Lösungsmittel am Rotationsverdampfer abgezogen. Der Rückstand wurde säulenchromatographisch aufgetrennt (Petrolether : Ethylacetat = 20:1). Es ergaben sich 1.42 g (70% d. Th.) an Diethylbis(2-phenyl-1,3-dithian-2-yl)germanium als farbloser Feststoff. DC (Petrolether : Ethylacetat = 20:1): R_{f}=0.51
Schmelzpunkt: 112-115 °C
¹H-NMR (200 MHz; CDCl₃): δ (ppm): 7.82-7.86 (m, 4H, Ar-H^{2,6}), 7.01-7.24 (m, 6H, Ar-H^{3,4,5}), 2.55 - 2.69 (m, 4H, S-CH₂-), 2.12 - 2.23 (m, 4H, S-CH₂-), 1.63 - 2.01 (m, 4H -CH₂-), 1.19 (m, 4H, Ge-CH2-), 1.02 (m, 6H -CH₃).
¹³C-NMR (200 MHz; CDCl₃): δ (ppm): 140.58 (Ar-C¹), 130.38 (Ar-C⁴), 128.18 (Ar-C^{2,6}), 125.53 (Ar-C^{3,5}), 51.90 (Ge-C-S), 25.88 (S-CH₂-), 25.16 (-CH₂-), 10.26 (Ge-CH₂-), 4.74 (-CH₃).

### Beispiel 3: Synthese von Bisbenzoyldiethylgermanium

In einem 25 ml Rundkolben wurden 1.12 g (2.24 mmol) Diethyl-bis(2-phenyl-1,3-dithian-2-yl)germanium vorgelegt, und in 15 ml wässrigem THF (THF:Wasser 4:1) gelöst. Nach der Zugabe von 3.53 g (35.01 mmol) CaCO₃ wurde die Suspension 5 min bei Raumtemperatur gerührt. Unter Lichtschutz wurden 6.83 g (26.93 mmol) Iod portionsweise zugegeben. Nach 3 h Rühren bei Raumtemperatur wurde das Reaktionsgemisch mit 15 ml Diethylether verdünnt, und überschüssiges Iod durch Zugabe von 20 ml einer gesättigten Na₂S₂O₄-Lösung unter starkem Rühren zersetzt. Die dabei enstandenen Salze wurden durch Filtration über Hyflo von der Reaktionslösung abgetrennt, und mit Diethylether (3 x 15 ml) gewaschen. Die vereinten organischen Phasen wurden mit Na₂SO₄ getrocknet, abfiltriert, und das Lösungsmittel am Roationsverdampfer abgezogen. Der Rückstand wurde säulenchromatographisch (Petrolether : Ethylacetat 20:1) aufgetrennt. Es ergaben sich 0.46 g (60% d. Th.) an Bisbenzoyldiethylgermanium als gelber Feststoff. DC (Petrolether : Ethylacetat = 20:1): R_{f}=0.42
UV-VIS: λₘₐₓ: 418,5 nm, ε = 4880 dm²/mol
¹H-NMR (200 MHz; CDCl₃): δ (ppm): 7.70-7.75 (m, 2H, Ar-H^{2,6}), 7.37-7.50 (m, 3H, Ar-H^{3,4,5}), 1.50 (d, 4H, -CH₂), 1.11 (t, 6H, -CH₃).
¹³C-NMR (200 MHz; CDCl₃) : δ (ppm): 230.22 (-C=O), 141.23 (Ar-C¹), 133. 66 (Ar-C⁴), 129.06 (Ar-C^{2,6}), 128.720 (Ar-C^{3,5}), 9.11 (-CH₂), 6.61 (-CH₃) .
IR (cm⁻¹): 2959, 2911 , 1622 (C=O), 1579, 1447, 1308, 1207, 1169, 1022, 892, 767, 688

Im Vergleich zu langwellig absorbierenden Norrish-Typ-I-Photoinitiatoren (= Photoinitiatoren, deren monomolekulare Photolyse direkt polymerisationsauslösende Radikale liefert), wie z.B. das kommerzielle Bisacylphosphinoxid Irgacure 819 (Bis(2,4,6-trimethylbenzoyl)-phenyl-phosphinoxid) mit dem langwelligsten Absorptionsmaxima bei 397 nm, liegt das Maximum des Norrish-Typ-I-Photoinitiators Benzoyltrimethylgermanium mit 411,5 nm bzw. das Maximum von Bisbenzoyldiethylgermanium mit 418.5 nm deutlicher bathochromer, was die Durchhärtungstiefe der Photopolyreaktionsprodukte signifikant verbessert. Einzig die Gruppe der spaltenden Titanocene haben ein Maximum bei etwa 480 nm, allerdings weisen diese bekannterweise (K. Dietliker; Photoinitiators for Free Radical, Cationic and Anionic Photopolymerization 2nd Ed. Sita Technology Ld, London UK p.228-239) keine ausreichenden Photobleachingeffekt auf, was zu orange gefärbten Polymeren führt.

Im Vergleich zum im Dentalbereich weit verbreitet verwendeten Norrish-Typ-II-Photoinitiator Campherchinon (λₘₐₓ: 468 nm), der zur effizienten Radikalbildung ein zusätzliches Reduktionsmittel benötigt, liegt das Absorptionsmaximum von Benzoyltrimethylgermanium deutlich kurzwelliger und zeigt ein sehr gutes Ausbleichen (Photobleaching) beim Bestrahlen.

### Beispiel 4: Herstellung eines Kompositzements unter Verwendung des Benzoyltrimethylgermaniums aus Beispiel 1

Entsprechend der nachfolgend aufgeführten Tabelle 1 wurden Kompositbefestigungszemente auf der Basis einer Methacrylatmischung und unter Einbeziehung entweder unterschiedlicher Konzentrationen des Benzoyltrimethylgermaniums aus Beispiel 1 (Zement A bis C) oder einer Mischung aus Campherchinon und p-N,N-Dimethylaminobenzoesäureethylester (Zement D, Vergleich) mittels eines Walzenstuhles (Modell "Exakt", Exakt Apparatebau, Norderstedt) hergestellt. Dabei enthielten die Zemente B bzw. D die gleiche molare Konzentration an Photoinitiator, d.h. an Benzoyltrimethylgermanium (Zement B) bzw. Campherchinon (Zement D) Von den Materialien wurden Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und der Exothermzeit. Zusätzlich wurde die Gelbfärbung der nichtausgehärteten Pasten und sowie der ausgehärteten Zemente entsprechend der DIN-Norm 5033 "Farbmessung" unter Verwendung des L*a*b*-Farbmesssystems Minolta CR-300 anhand des b*-Wertes charakterisiert, wobei weiterhin ein b*-Wert von -2,7 für Zementpasten-Rezeptur ohne Initiatorkomponenten gemessen wurde.

**Tabelle 1: Zusammensetzung der Kompositzemente (Angaben in Gew.-%)**

| **Komponente** | **Zement A** | **Zement B** | **Zement C** | **Zement D²)** |
|---|---|---|---|---|
| Benzoyltrimethylgermanium | 0,10 | 0,32 | 0,50 | - |
| Campherchinon | - | - | - | 0,24 |
| p-N,N-Dimethylaminobenzoesäureethylester | - | - | - | 0,23 |
| UDMA¹⁾ | 32,11 | 31,89 | 31,71 | 31,80 |
| Triethylenglycoldimethacrylat | 7,81 | 7,81 | 7,81 | 7,81 |
| Aerosil OX-50 (Degussa) | 41,27 | 41,27 | 41,28 | 41,23 |
| Ytterbiumtrifluorid (Rhone-Poulenc) | 18,71 | 18,71 | 18,70 | 18,69 |

| | | | | |
|---|---|---|---|---|
| ¹⁾ Additionsprodukt aus 2 mol 2-Hydroxyethylmethacrylat und 1 mol 2,2,4-Trimethylhexamethylendiisocyanat ²⁾ Vergleich | | | | |

**Tabelle 2: Eigenschaften der Kompositzemente**

| **Komponente** | **Zement A** | **Zement B** | **Zement C** | **Zement D²⁾** |
|---|---|---|---|---|
| Exothermzeit (s) | 13 | 12 | 11 | 8 |
| Biegefestigkeit (MPa) nach 24 h WL¹⁾ | 102 | 116 | 129 | 118 |
| E-Modul (MPa) nach 24 h WL¹⁾ | 3230 | 5240 | 5560 | 5580 |
| b*-Wert Paste vor Aushärtung | 7,7 | 16,0 | 19,6 | 27,4 |
| b*-Wert Zement nach Aushärtung | -5,8 | -1,9 | 0,7 | 4,5 |

| | | | | |
|---|---|---|---|---|
| ¹⁾ WL = Wasserlagerung der Prüfkörper bei 37 °C ²⁾ Vergleich | | | | |

Aus Tabelle 2 ist ersichtlich, daß die auf Benzoyltrimethylgermanium basierende Zemente mit zunehmender Photoinitiatorkonzentration eine kürzere Exothermzeit und damit eine schnellere Aushärtung ergeben und im Vergleich zum Zement D (konventionelle Photoinitiatormischung auf der Basis einer Mischung aus Campherchinon und p-N,N-Dimethylaminobenzoesäureethylester) ab einer Konzentration von 0,32 Gew.-% Benzoyltrimethylgermanium (Zement B) Materialien mit vergleichbaren mechanischen Eigenschaften erhalten werden. Überraschenderweise wurde gefunden, daß die ausgehärteten Zemente auf der Basis von Benzoyltrimethylgermanium negative oder nur kleine positive b*-Werte und damit keine Gelbverfärbung zeigen, während sich für den ausgehärteten Zement auf der Basis von Campherchinon ein b*-Wert von 4,5 ergab, was einer deutlichen Gelbverfärbung entspricht.

### Beispiel 5: Herstellung eines Füllungskomposits unter Verwendung des Benzoyltrimethylgermaniums aus Bsp. 1

Entsprechend der nachfolgend aufgeführten Tabelle 3 wurde ein Füllungskomposit auf der Basis einer Methacrylatmischung und unter Einbeziehung entweder unterschiedlicher Konzentrationen des Benzoyltrimethylgermaniums aus Beispiel 1 (Komposit E) oder einer Mischung aus Campherchinon und p-N,N-Dimethylaminobenzoesäureethylester (Komposit F, Vergleich) mittels eines Kneters (Typ LPM 0.1 SP, Linden, Marienheide) hergestellt. Von den Materialien wurden analog Beispiel 4 Prüfkörper hergestellt und ausgehärtet. Nach der ISO-Norm ISO-4049 erfolgte die Bestimmung der Biegefestigkeit, des Biege-E-Moduls und des Polymeriationsschrumpfes.

**Tabelle 3: Zusammensetzung der Füllungskomposite (Angaben in Gew.-%)**

| **Komponente** | **Komposit E** | **Komposit F⁵⁾** |
|---|---|---|
| Monomerharz¹⁾ | 18,06 | 17,99 |
| Benzoyltrimethylgermanium | 0,08 | - |
| Campherchinon | - | 0,05 |
| p-N,N-Dimethylaminobenzoesäureethylester | - | 0,09 |
| Glasfüller GM27884 (Schott)²⁾ | 51,6 | 51,61 |
| Sphärosil (Tokoyama Soda)³⁾ | 14,37 | 14,36 |
| Ytterbiumtrifluorid (Rhone-Poulenc) | 14,89 | 14,89 |
| OX-50⁴⁾ | 0,2 | 0,2 |

| | | |
|---|---|---|
| ¹⁾ Mischung aus 42.4 Gew.-% Bis-GMA, 37,4 Gew.-% UDMA und 20,2 Gew.-% Triethylenglycoldimethacrylat ²⁾ Silanisierter Ba-Al-Borosilikatglasfüller mit einer mittleren Partikelgröße von 1,5 µm, ³⁾ SiO₂-ZrO₂-Mischoxid, mittl. Primärpartikelgröße: 250 nm ⁴⁾ Silanisiertes pyrogenes SiO₂ OX-50 (Degussa) ⁵⁾ Vergleich | | |

**Tabelle 4: Eigenschaften der Füllungskomposite**

| **Materialeigenschaft** | **Komposit E** | **Komposit F²⁾** |
|---|---|---|
| Exothermzeit (s) | 10 | 9 |
| Biegefestigkeit (MPa) nach 24 h WL¹⁾ | 150 | 168 |
| Biege-E-Modul (GPa) nach 24 h WL¹⁾ | 10540 | 12190 |

| | | |
|---|---|---|
| ¹⁾ WL = Wasserlagerung der Prüfkörper bei 37 °C ²⁾ Vergleich | | |

### Beispiel 6: Herstellung eines stark aciden Kompositzementes unter Verwendung des Benzoyltrimethylgermaniums aus Beispiel 1

Entsprechend der nachfolgend aufgeführten Tabelle 5 wurden Kompositbefestigungszemente auf der Basis einer Mischung von zwei Dimethacrylaten mit der aciden Phosphonsäure MA-154 (2-[2-Dihydroxyphosphoryl)-ethoxymethyl]-acrylsäureethylester und unter Einbeziehung entweder des Benzoyltrimethylgermaniums aus Beispiel 1 (Zement G) oder einer Mischung aus Campherchinon und p-N,N-Dimethylaminobenzoesäureethylester (Zement H, Vergleich) mittels eines Walzenstuhles (Modell "Exakt", Exakt Apparatebau, Norderstedt) hergestellt. Von den Materialien wurden analog Beispiel 2 Prüfkörper präpariert, ausgehärtet und die Biegefestigkeit der E-Moduls bestimmt.

**Tabelle 5: Zusammensetzung der aciden Kompositzemente (Angaben in Gew.-%)**

| **Komponente** | **Zement G** | **Zement H²⁾** |
|---|---|---|
| Benzoyltrimethylgermanium | 0,33 | - |
| Campherchinon | - | 0,24 |
| p-N,N-Dimethylaminobenzoesäureethylester | - | 0,23 |
| UDMA¹⁾ | 21,88 | 21,82 |
| Triethylenglycoldimethacrylat | 7,81 | 7,81 |
| Phosphonsäure MA-154 | 10,01 | 9,99 |
| Aerosil OX-50 (Degussa) | 41,27 | 41,22 |
| Ytterbiumtrifluorid (Rhone-Poulenc) | 18,70 | 18,69 |

| | | |
|---|---|---|
| Additionsprodukt aus 2 mol 2-Hydroxyethylmethacrylat und 1 mol 2,2,4-Trimethylhexamethylendiisocyanat Vergleich ¹⁾ WL = Wasserlagerung der Prüfkörper bei 37 °C ²⁾ Vergleich | | |

**Tabelle 6: Eigenschaften der Kompositzemente**

| **Komponente** | **Zement G** | **Zement H²⁾** |
|---|---|---|
| Biegefestigkeit (MPa) nach 24 h WL¹⁾ | 118 | 120 |
| E-Modul (MPa) nach 24 h WL¹⁾ | 5380 | 5690 |

| | | |
|---|---|---|
| ¹⁾ WL = Wasserlagerung der Prüfkörper bei 37 °C ²⁾ Vergleich | | |

Aus Tabelle 6 ist ersichtlich, daß der auf Benzoyltrimethylgermanium basierende Zement G im Vergleich zum Zement H (konventionelle Photoinitiatormischung einer Mischung aus Campherchinon und p-N,N-Dimethylaminobenzoesäureethylester) auch in Gegenwart von stark aciden Monomeren zu Materialien mit vergleichbaren mechanischen Eigenschaften führt.

### Beispiel 7: Vergleich der Aktivität der Acylgermane mit bekannten Photoinitiatoren, die im sichtbaren Bereich absorbieren.

Die Aktivität der Photoinitiatoren wurde mittels Photo-DSC (Differential Scanning Calorimetry) Messungen an einem DSC-50 Gerät der Fa. Shimadzu gemessen, wobei die Proben wahlweise mit verschiedenen Dentallampen bestrahlt wurden (Astalis 3: Halogenlampe, Wellenlängenbereich 400-500 nm, Intensität 530 mW/cm²; Bluephase C8: LED, Wellenlängenbereich 430-490 nm, Intensität 1100 mW/cm²; Ivoclar Vivadent AG). Gekennzeichnet wird die Aktivität durch den Zeitpunkt des Peakmaximums (tₘₐₓ), die Polymerisationsrate (R_{P}), welche der Peakhöhe entspricht, und den Doppelbindungsumsatz (DBC). Die jeweiligen Photoinitiatoren wurden in einer Harzmischung aus D₃MA:UDMA:Bis-GMA = 1:1:1 gelöst, und anschließend in einem Aluminiumtiegel mittels DSC gemessen.

Die Tabellen 7 und 8 zeigen Photo-DSC-Daten von CQ (Campherquinon / Dimethylaminobenzoesäureethylester), Irg 819 (Bis(2,4,6-trimethylbenzoyl)phenylphosphinoxid), Benzoyltrimethylgermanium (Mono-AG) und Bisacyldiethylgermanium (Bis-AG) bei einer Konzentration von 0,022 mmol PI (Photoinitiator) pro Gramm Harz.

Tabelle 9 zeigt Photo-DSC-Daten von Irg 819 (Bis(2,4,6-trimethylbenzoyl)phenylphosphinoxid), Benzoyltrimethylgermanium (Mono-AG) und Bisacyldiethylgermanium (Bis-AG) bei unterschiedlichen PI Konzentrationen.

**Tabelle 7: Bestrahlung mit einer Astralis 3-Lampe (400-500 nm)**

| **PI** | **tₘₐₓ** [s] | **R_{P}**x10⁻³ [mol/(lxs)] | **DBC [%]** |
|---|---|---|---|
| CQ | 14 | 54.9 | 59 |
| Irg 819 | 11 | 67.5 | 59 |
| Mono-AG | 16 | 51.0 | 56 |
| Bis-AG | 7.8 | 99.2 | 84 |

**Tabelle 8: Bestrahlung mit Bluephase C8-Lampe (430-490 nm)**

| **PI** | **tₘₐₓ** [s] | **R_{P}**x10⁻³ [mol/(lxs)] | **DBC [%]** |
|---|---|---|---|
| CQ | 13 | 59.9 | 61 |
| Irg 819 | 17 | 43.5 | 45 |
| Momo-AG | 16 | 48.3 | 49 |
| Bis-AG | 7.8 | 98.5 | 72 |

**Tabelle 9: Aktivitätsvergleich unterschiedlicher Initiatoren**

| **Konzentration [mmol/g]** | **PI** | **tₘₐₓ** [s] | **R_{P}**x10**⁻³** [mol/(lxs)] | **DBC [%]** |
|---|---|---|---|---|
| 0.0055 | Irg 819 | 14 | 48.3 | 48 |
| | Mono-AG | 24 | 36.2 | 48 |
| | Bis-AG | 9 | 85.6 | 66 |
| 0.0014 | Irg 819 | 23 | 30.4 | 42 |
| | Mono-AG | 52 | 16.4 | 30 |
| | Bis-AG | 12 | 61.0 | 56 |

## Patentansprüche

1. Zusammensetzung mit mindestens einem polymerisierbaren Bindemittel und einem Polymerisationsinitiator, **dadurch gekennzeichnet, daß** sie mindestens ein Acylgerman gemäß der allgemeinen Formel (I) enthält, 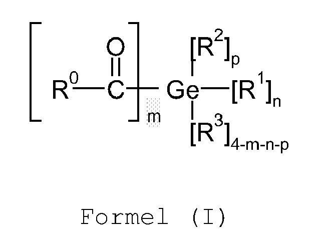 in der
R⁰ C₁₋₁₈-Alkyl, C₂₋₁₈-Alkenyl, wobei diese Reste unsubstituiert oder einfach oder mehrfach durch Halogen substituiert sein können, -OR¹⁰, -OCO-R¹⁰, -OCO-Hal, -COO-R¹⁰, -CH=CH-CO-OR¹⁰, -N (R¹¹) -CO-R¹⁰, -N (R¹¹) -CO-Hal, -C(C₁₋₄-Alkyl)=C(C₁₋₄-Alkyl)-CO-OR¹⁰, -CO-NR¹¹R¹², -CH=CH-Phenyl, -C(C₁₋₄-Alkyl)=C(C₁₋₄-Alkyl)-Phenyl, C₃₋₁₂-Cycloalkyl, C₂₋₁₈-Alkenyl, Phenyl-C₁₋₄-Alkyl, Phenyl, Naphtyl, Antryl, Biphenyl, ein 5- oder 6-gliedriger O-, S- oder N-haltiger heterocyclischer Ring, wobei alle genannten Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder 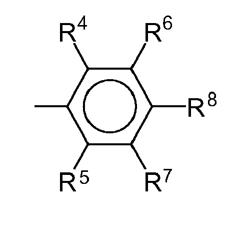 ist, wobei
R¹⁰ H, C₁₋₁₈-Alkyl, C₂₋₁₈-Alkenyl, C₂₋₁₈-Alkenyl, das durch ein oder mehrere Sauerstoffatome unterbrochen ist, ein Di-, Tri-, Tetra- oder Polyethylenglycolrest, C₃₋₁₂-Cycloalkyl, Tetrahydropyran-2-yl, Phenyl-C₁₋₄-Alkylen, Phenyl-C₁₋₄-Alkenylen, C₁₋₆-Alkyl, das unsubstituiert oder durch Halogen, Cyclohexyl, Cylopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können,
R¹¹, R¹² unabhängig voneinander H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloakyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxyund/oder C₁₋₈-Alkylthioreste substituiert sein können, oder R¹¹ und R¹² bilden zusammen einen 5- oder 6-gliedrigen O-, S- oder N-haltigen heterocyclischen Ring, der seinerseits mit einem aliphatischen oder aromatischen Ring anelliert sein kann,
R¹, R² unabhängig voneinander H oder 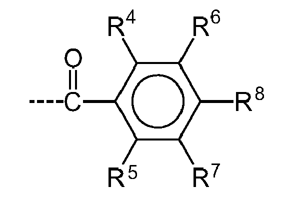 sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆-Alkylrest sind;
R⁶, R⁷, R⁸ unabhängig voneinander jeweils H, Halogen, ein verzweigter, zyklischer oder vorzugsweise linearer C₁₋₂₀-Alkyl-, -Alkenyl-, -Alkyloxy- oder -Alkenoxy-Rest sind, der durch ein oder mehrere O, S oder - NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, -[Si(CH₃)₂]_{y}-CH₃ mit y = 1 bis 20 ist und
R²⁰ H, Halogen, ein verzweigter, zyklischer oder vorzugsweise linearer C₁₋₂₀-Alkyl-, -Alkenyl-, -Alkyloxy- oder -Alkenoxy-Rest ist;
R³ ein verzweigter oder vorzugsweiser linearer C₁₋₁₈-Alkyl-Rest oder C₂₋₁₈-Alkenyl-Rest ist, wobei diese Reste unsubstituiert oder einfach oder mehrfach durch einen Rest substituiert sein können, der aus der folgenden Gruppe ausgewählt ist: Halogen, CN, 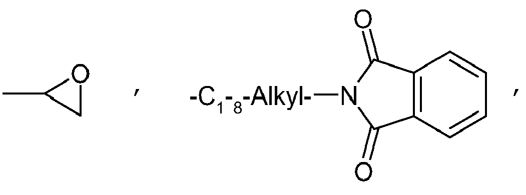 -OR¹⁰, -SR¹⁰, -OCO-R¹⁰, -COO-R¹⁰, -CH=CH-CO-OR¹⁰, -C(C₁₋₄--Alkyl)=C(C₁₋₄-Alkyl)-CO-OR¹⁰, -CO-R¹³, -CO-CH=CH-CO-C₁₋₆-Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N (R¹¹) -CO-NR¹¹R¹², -N (R¹¹) -CO-Hal, -CO-NR¹¹R¹², SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄-Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein 5- oder 6-gliedriger O-, S- oder N-haltiger heterocyclischer Ring, Benzophenonyl, Thisanthonyl, wobei
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈-Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei
R¹⁷, R¹⁸, R¹⁹ unabhängig voneinander jeweils H, C₁₋₈-Alkyl, C₂₋₈-Alkenyl, C₇₋₉-Phenylalkyl, -O-C₁₋₈-Alkyl oder Phenyl sind, und
wobei R¹⁰, R¹¹ und R¹² wie oben definiert sind;
oder
R³ ist ein verzweigter oder vorzugsweiser linearer C₂-₁₈-Alkyl-Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch einen Rest substituiert sein können, der aus der folgenden Gruppe gewählt ist:
Halogen, CN, -OR¹⁰, -SR¹⁰, -OCO-R¹⁰, -COO-R¹⁰, -NR¹¹R¹², -N(R¹¹) -CO-R¹⁰, -N(R¹¹) -COO-R¹⁰, -N(R¹¹) -CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, Phenyl-C₁₋₄-Alkyl, Phenyl, C₅₋₁₂-Cycloalkyl;
wobei R¹⁰, R¹¹ und R¹² wie oben definiert sind;
oder
R³ ist ein verzweigter oder vorzugsweiser linearer C₂-₁₈-Alkyl-Rest oder ein C₂₋₁₈-Alkylen-Rest, der einfach oder mehrfach durch -CO-, -COO-, -OCO-, -OCOO-, -CO-N(R¹¹)-, -N(R¹¹)-CO-, -N(R¹¹)-CO-N(R¹¹)-, -N(R¹¹)-COO-, -COO-C₁₋₆-Alkylen, -COS-C₁₋₁₈-Alkylen, -SO₂-, -SO₂-O-, -SO₂-N(R¹¹)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloakylen oder einen 5- oder 6-gliedrigen O-, S- oder N-haltigen heterocyclischen Ring unterbrochen ist;
wobei R¹¹ wie oben definiert ist;
oder
R³ ist Trimethylsilyl, Hal- (CH₃) ₂Si- [OSi (CH₃) ₂] ᵣ-, (CH₃)₃Si-[OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
R³ ist Phenyl-C₁₋₄-Alkyl, Phenyl, Naphthyl oder Biphenyl, C₅₋₁₂-Cycloalkyl oder ein 5- oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substituiert sein können,
wobei R¹¹ und R¹² wie oben definiert sind,
m 1, 2 oder 3 ist,
n 0 oder 1 ist,
p 0 oder 1 ist;
oder
R³ ist Halogen, OH, ein aromatischer C₆₋₃₀-Rest, der durch einen verzweigten, zyklischen oder vorzugsweise linearen C₁₋₂₀-Alkyl-, -Alkenyl-, -Alkoxy- oder -Alkenoxy-Rest substituiert sein kann, wobei die genannten Reste ein- oder mehrfach durch O, S oder -NR²⁰- unterbrochen und/oder durch eine oder mehrere polymerisationsfähige Gruppen und/oder Reste R⁹ substituiert sein können, oder ein verzweigter, zyklischer oder vorzugsweise linearer C₁₋₂₀-Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann, wobei R⁹ und R²⁰ wie oben definiert sind.

2. Zusammensetzung mit mindestens einem polymerisierbaren Bindemittel und einem Polymerisationsinitiator, die mindestens ein Acylgerman gemäß der allgemeinen Formel (II) enthält, 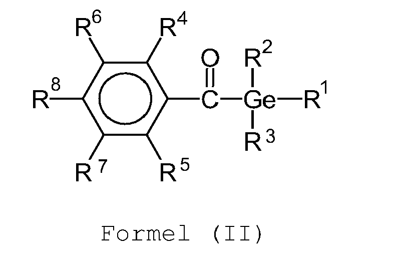 in der
R¹, R² unabhängig voneinander 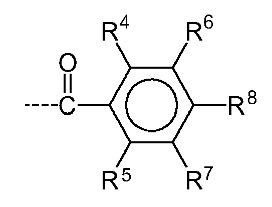 oder H sind, oder eine der für R³ angegebenen Bedeutungen haben;
R³ Halogen, OH, ein aromatischer C₆₋₃₀-Rest, der durch einen verzweigten, zyklischen oder vorzugsweise linearen C₁₋₂₀-Alkyl-, -Alkenyl-, -Alkoxy- oder -Alkenoxy-Rest substituiert sein kann, wobei die genannten Reste ein- oder mehrfach durch O, S oder -NR²⁰- unterbrochen und/oder durch eine oder mehrere polymerisationsfähige Gruppen und/oder Reste R⁹ substituiert sein können, oder ein verzweigter, zyklischer oder vorzugsweise linearer C₁₋₂₀-Alkyl-, -Alkenyl-, -Alkoxy- oder -Alkenoxy-Rest ist, der ein- oder mehrfach durch O, S oder - NR²⁰- unterbrochen und durch eine oder mehrere polymerisationsfähige Gruppen und/oder Reste R⁹ substituiert sein kann;
R⁴, R⁵ unabhängig voneinander jeweils H, Halogen, ein linearer oder verzweigter C₁₋₆-Alkyl- oder -O-C₁₋₆-Alkylrest sind;
R⁶, R⁷, R⁸ unabhängig voneinander jeweils H, Halogen, ein verzweigter, zyklischer oder vorzugsweise linearer C₁₋₂₀-Alkyl-, -Alkenyl-, -Alkyloxy- oder -Alkenoxy-Rest sind, der ein- oder mehrfach durch O, S oder -NR²⁰- unterbrochen und durch eine oder mehrere polymerisationsfähige Gruppen und/oder Reste R⁹ substituiert sein kann;
R⁹ -OH, -CₓF₂ₓ₊₁ mit x = 1 bis 20, -[Si(CH₃)₂]_{y}-CH₃ mit y = 1 bis 20 ist; und
R²⁰ H, Halogen, ein verzweigter, zyklischer oder vorzugsweise linearer C₁₋₂₀-Alkyl-, -Alkenyl-, -Alkyloxy- oder -Alkenoxy-Rest ist.

3. Zusammensetzung nach Anspruch 1 oder 2, bei der die polymerisationsfähigen Gruppen aus Vinyl, Styryl, (Meth)acrylat, (Meth)acrylamid oder N-Alkylacrylamid ausgewählt sind.

4. Zusammensetzung nach einem der vorhergehenden Ansprüche, bei der die Reste R², R³, R⁶, R⁷ und R⁸ jeweils mit 1 bis 3 polymerisierbaren Gruppe substituiert sind.

5. Zusammensetzung nach einem der vorhergehenden Ansprüche, die bezogen auf die Gesamtmasse der Zusammensetzung 0,001 bis 5 Gew.-% des Acylgermans der Formel (I) enthält.

6. Zusammensetzung nach einem der vorhergehenden Ansprüche, die als polymerisierbares Bindemittel mindestens ein radikalisch polymerisierbare Monomer und/oder Prepolymer enthält.

7. Zusammensetzung nach Anspruch 6, die als Bindemittel ein mono- oder multifunktionelles (Meth)acrylat oder eine Mischung davon enthält.

8. Zusammensetzung nach Anspruch 6 oder 7, die mindestens ein radikalisch ringöffnend polymerisierbares Monomer enthält.

9. Zusammensetzung nach einem der vorhergehenden Ansprüche, die als Bindemittel eine Mischung von mono- und/oder multifunktionellen Mercaptoverbindungen und di- und/oder multifunktionellen ungesättigten Monomeren enthält.

10. Zusammensetzung nach einem der vorhergehenden Ansprüche, die mindestens einen weiteren Initiator für die radikalische Polymerisation enthält.

11. Zusammensetzung nach einem der vorhergehenden Ansprüche, die zusätzlich Füllstoff enthält.

12. Zusammensetzung nach einem der vorhergehenden Ansprüche, die zusätzlich mindestens ein Additiv enthält, das aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufheller, Fließverbesserern, Verdickern und Antischaummitteln ausgewählt ist.

13. Zusammensetzung, die
0,001 bis 5 Gew.-% Acylgerman gemäß Formel (I),
5 bis 99,9 Gew.-% polymerisierbares Bindemittel,
0 bis 90 Gew.-% Füllstoff enthält, jeweils bezogen auf die Gesamtmasse der Zusammensetzung.

14. Zusammensetzung nach Anspruch 13, die 0 bis 50 Gew.-% weiteres Additiv enthält.

15. System zur Herstellung von Formkörpern, daß eine einer Zusammensetzung gemäß einem der Ansprüche 1 bis 14 und einer LED-Lichtquelle umfaßt.

16. System nach Anspruch 15, bei dem die LED-Lichtquelle eine Wellenlänge im Bereich von 400 bis 550 nm hat und das Acylgerman eine Aktivierungswellenlänge im Bereich von 400 bis 550 nm aufweist.

17. Verwendung eines Acylgermans gemäß Formel (I) als Initiator für die radikalische Polymerisation.

18. Verwendung eines Acylgermans gemäß Formel (I) zur Herstellung von Adhäsiven, Beschichtungen, Zementen, Kompositen, Formteilen oder Dentalwerkstoffen.

19. Verfahren zur Herstellung eines Formkörpers, bei dem man eine Zusammensetzung gemäß einem der Ansprüche 1 bis 14 zu einem Körper mit der gewünschten Form formt und anschließend ganz oder teilweise härtet.

20. Verfahren nach Anspruch 20 zur Herstellung von dentalen Kronen, Brücken, Inlays oder künstlichen Zähnen.
